# EUROPÄISCHE PATENTSCHRIFT

(11) **EP 2 211 976 B1**
(45) Veröffentlichungstag und Bekanntmachung des Hinweises auf die Patenterteilung: **30.03.2016**
(21) Anmeldenummer: 08844073.0
(22) Anmeldetag: 24.10.2008
(51) Int. Cl.: A61M 39/12, F16L 33/22, A61M 39/10, A61M 1/06

(54) **VORRICHTUNG ZUM ANSCHLIESSEN EINES SAUGSCHLAUCHS**
DEVICE FOR CONNECTING A SUCTION HOSE
DISPOSITIF DE RACCORDEMENT D'UN CONDUIT SOUPLE D'ASPIRATION

(30) Priorität: 30.10.2007 CH 16802007
(43) Veröffentlichungstag der Anmeldung: 04.08.2010
(73) Patentinhaber: Medela Holding AG, 6340 Baar (CH)
(72) Erfinder: KÜNZLER, Hansruedi, CH-8932 Mettmenstetten (CH); STADELMANN, Urs, CH-6004 Luzern (CH); VISCHER, Peter, CH-6403 Küssnacht am Rigi (CH)
(74) Vertreter: Clerc, Natalia
(86) Internationale Anmeldenummer: PCT/CH2008/000447
(87) Internationale Veröffentlichungsnummer: WO 2009/055949

(56) Entgegenhaltungen:
- EP-A- 1 468 705
- EP-A- 1 902 747
- WO-A-2006/004943
- DE-U1- 8 804 038
- DE-U1- 20 214 608
- DE-U1-202005 017 522
- US-A- 4 749 217
- US-A- 5 868 435
- US-B1- 6 641 177

## Beschreibung

### Technisches Gebiet

Die Erfindung betrifft eine Vorrichtung zum Anschliessen eines Saugschlauchs gemäss Oberbegriff des Patentanspruchs 1, eine Verbindungseinheit gemäss Oberbegriff des Patentanspruchs 10 und eine Saugeinheit gemäss Oberbegriff des Patentanspruchs 14.

### Stand der Technik

Im Stand der Technik ist eine Vielzahl von unterschiedlichsten Schlauchverbindungen bekannt, insbesondere für Saug- und Drainageschläuche im medizinaltechnischen Bereich. Beispiele hierfür sind in EP 1 552 858, US 3 876 234, US 4 123 091, US 4 511 163, US 4 607 868, US 5 988 700 und WO 98/24500 beschrieben.

Üblicherweise wird bei einer Schlauchverbindung bzw. einer Vorrichtung zum Anschliessen eines Schlauchs der Schlauch auf oder in einen Anschlussstutzen geschoben und dank der Form des Anschlussstutzens oder mittels eines zusätzlichen Befestigungsteils fixiert.

Bei den meisten Vorrichtungen lassen sich die Saugschläuche auf einfache Art und Weise ersetzen. Es gibt jedoch Anwendungsbereiche, beispielsweise in Drainageanwendungen wie die Thoraxdrainage oder bei Brustpumpen zum Abpumpen von menschlicher Muttermilch, bei welchen es nicht erwünscht ist, dass der Saugschlauch von der Vorrichtung entfernt und evtl. durch einen anderen ersetzt werden kann. Einige Gründe hierfür sind Hygiene, Funktionssicherheit des Pumpsystems und Schutz vor Billigkopien bzw. mangelhaften Kopien von Ersatzteilen.

DE 88 04 038 U offenbart ein Verbindungsstück für einen Schlauch mit einer muffenartigen Sicherungshülse, welche auf das Schlauchende aufgeschoben wird, so dass das Schlauchende zwischen der Hülse und dem Verbindungsstück festgeklemmt wird.

DE 20 2005 017 522 U zeigt eine Rohrverbindung für Wasserinstallationen mit einem Grundkörper und einer Schiebhülse. Der Grundkörper weist einen Einrastzahn und die Schiebehülse eine Einrastrille auf, die ineinander verhakt werden.

US 6 641 177 offenbart ein Kopplungssystem für einen Schlauch. Dieses Kopplungssystem wird in Whirlpools und Badewannen verwendet. Das Kopplungssystem weist eine Hülse auf, welche eine oktagonale Form haben kann.

DE 202 14 608 U1 offenbart ebenfalls ein Kopplungssystem für einem Schlauch, mit einer Hülse, welche eine hexagonale Außenkontur besitzen kann.

WO 2006/004943 zeigt eine Schlauchverbindung für ein System zur künstlichen Ernährung. Ein Schlauchende wird über einen Stutzen eines Grundelements gestülpt. Darüber wird eine zweiteilige Hülse aufgeklippt. Die zwei Teile werden hierfür um das Grundelement gelegt und dann miteinander verbunden werden. Es können bekannte Verriegelungsmittel verwendet werden.

EP 1 902 747 wurde erst nach dem Prioritätsdatum dieser Anmeldung veröffentlicht. Es wird ein trennbares Schlauchverbindungssystem beschrieben, das die Verwendung vom unzulässigen Verbindungen oder Steckern verhindern soll.

### Darstellung der Erfindung

Es ist deshalb eine Aufgabe der Erfindung, eine Vorrichtung zum Anschliessen eines Saugschlauchs zu schaffen, welche ein zerstörungsfreies Entfernen des Saugschlauchs und ein Ersetzen des Saugschlauchs durch einen anderen Schlauch verhindert.

Diese Aufgabe lösen eine Vorrichtung mit den Merkmalen des Patentanspruchs 1 sowie eine Verbindungseinheit und eine Saugeinheit mit den Merkmalen des Patentanspruchs 10 bzw. 14.

Die erfindungsgemässe Vorrichtung zum Anschliessen eines Saugschlauchs weist einen Anschlussstutzen auf, welcher derart ausgebildet ist, dass der Saugschlauch dichtend über einen Bereich des Anschlussstutzens aufschiebbar ist. Die Vorrichtung umfasst ferner eine Hülse, welche über den genannten Bereich des Anschlussstutzens aufschiebbar ist und diesen mindestens teilweise überdeckt, wobei zwischen Hülse und Anschlussstutzen Raum für den Saugschlauch verbleibt. Die Verbindung zwischen Hülse und Anschlussstutzen ist nur durch Zerstörung mindestens eines der zwei Teile, vorzugsweise beider Teile, trennbar. Die Hülse weist eine äussere Oberflächengestaltung auf, welche ein dichtendes Anliegen eines zweiten Saugschlauchs auf der Hülse verhindert.

Dadurch lässt sich zum einen ein hersteller- bzw. werkseitig dichtend angeschlossener Schlauch nicht zerstörungsfrei entfernen und wieder aufsetzen. Es wird verhindert, dass die Vorrichtung oder der Schlauch unsachgemäss miteinander verbunden werden. Die Funktionstüchtigkeit der Vorrichtung und die Erfüllung von Hygienevorschriften sind gewährleistet. Es ist zudem sichergestellt, dass kein Billigschlauch anstelle des Originalschlauchs verwendet werden kann.

Des Weiteren kann ein herstellerseitig angebrachter Schlauch nicht einfach abgeschnitten und durch einen Ersatzschlauch mit einem grösseren Durchmesser ersetzt werden, welcher über die Hülse geschoben wird. Die äussere Form der Hülse verunmöglicht eine dichtende Verbindung mit einem derartigen Ersatzschlauch. Auch dies gewährleistet die Funktionssicherheit der Saugeinheit, da ein anderer Saugdurchmesser ein anderes Saugverhalten bedingen würde.

Zudem wird dadurch gewährleistet, dass die gesamten herstellerseitig miteinander verbundenen Teile der Vorrichtung nur solange verwendet werden können, wie vom Hersteller vorgesehen bzw. bis zur Verschmutzung oder einem Defekt eines einzelnen der Teile.

Des Weiteren wird verhindert, dass einfach herstellbare und mangelhafte Billigkopien für einzelne Teile der Vorrichtung angeboten werden können. Dies ist insbesondere im Drainage- und Brustpumpenbereich wesentlich, da dort die Brusthauben, Schläuche und andere patienten- bzw. mutterseitig benutzte Teile einer Saugeinheit Einmal-Gebrauchsgegenstände sind, in grossen Stückzahlen verwendet werden, aber trotzdem hohen Qualitätsansprüchen genügen müssen.

Erfindungsgemäß weist die Hülse einen Grundkörper mit einem äusseren Durchmesser und einen am Grundkörper angeformten äusseren Flansch mit einem grösseren Durchmesser als der genannte Durchmesser auf, wobei der Flansch mindestens teilweise umlaufend ausgebildet ist und eine von einer Kreisform abweichende Kontur aufweist.

Vorzugsweise sind Anschlussstutzen und Hülse aus Kunststoff, insbesondere im Spritzgussverfahren gefertigt. Dadurch lassen sich diese Teile kostengünstig herstellen.

Vorzugsweise sind Anschlussstutzen und Hülse über einen Schnappverschluss miteinander verbunden, so dass die Teile eine relativ einfache Form aufweisen und deshalb insbesondere im Spritzgussverfahren kostengünstig herstellbar sind.

Die Hülse liefert einen Beitrag an die Dichtung oder Fixierung des Saugschlauchs.

Die Hülse weist den weiteren Vorteil auf, dass sie den Schlauch in seinem Anschlussbereich gegen aussen schützt. Wenn zudem die Hülse dem Anschlussstutzen vorsteht, schützt sie den Saugschlauch in diesem Bereich vor einem Abknicken und möglichem Bruch.

Weitere vorteilhafte Ausführungsformen gehen aus den abhängigen Patentansprüchen hervor.

### Kurze Beschreibung der Zeichnungen

Im Folgenden wird der Erfindungsgegenstand anhand eines bevorzugten Ausführungsbeispiels, welches in den beiliegenden Zeichnungen dargestellt ist, erläutert. Es zeigen:
- Figur 1: eine perspektivische Darstellung einer Membrankappe einer Brustpumpe mit einer erfindungsgemässen Vorrichtung zum Anschliessen eines Saugschlauchs in Explosionsdarstellung;
- Figur 2: die Membrankappe mit der Vorrichtung gemäss Figur 1 im zusammengesteckten Zustand;
- Figur 3a: einen Längsschnitt durch die Kappe mit der Vorrichtung gemäss Figur 1;
- Figur 3b: den Längsschnitt gemäss Figur 3a mit einem aufgesetzten falschen Ersatzschlauch;
- Figur 4a: eine vergrösserte Darstellung eines Details gemäss Figur 3;
- Figur 4b: eine Variante des Details gemäss Figur 4a;
- Figur 5: eine vergrösserte Darstellung eines erfindungsgemässen Anschlussstutzens im Längsschnitt;
- Figur 6: einen Längsschnitt durch eine erfindungsgemässe Hülse;
- Figur 7: eine perspektivische Darstellung der Hülse gemäss Figur 6 von einer ersten Seite;
- Figur 8: eine perspektivische Darstellung der Hülse gemäss Figur 6 von einer zweiten Seite;
- Figur 9: einen Teil einer Brustpumpe mit Membrankappe und erfindungsgemässer Vorrichtung und
- Figur 10: eine perspektivische Darstellung eines Ersatzkits mit Membrankappe, erfindungsgemässer Vorrichtung, Brusthaube und Milchflasche.

### Wege zur Ausführung der Erfindung

Das nachfolgend beschriebene Ausführungsbeispiel bezieht sich auf eine Vorrichtung zum dichtenden Anschliessen eines Saugschlauchs an eine Membrankappe einer Brustpumpe zum Abpumpen von menschlicher Muttermilch. Eine derartige Membrankappe ist aus US 6 699 213 bekannt.

Die erfinderische Lehre ist jedoch nicht auf eine derartige Anwendung beschränkt. Die erfindungsgemässe Vorrichtung lässt sich im Bereich des technischen Gebietes von Brustpumpen beispielsweise auch bei anders gestalteten Membrankappen, bei Anschlüssen an die Pumpe selber und beim Anschluss an die Brusthaube verwenden. Des Weiteren lässt sie sich in analoger Weise im Bereich der Drainage, insbesondere der Wund- oder Thoraxdrainage oder in anderen Bereichen, insbesondere der Medizinaltechnik verwenden.

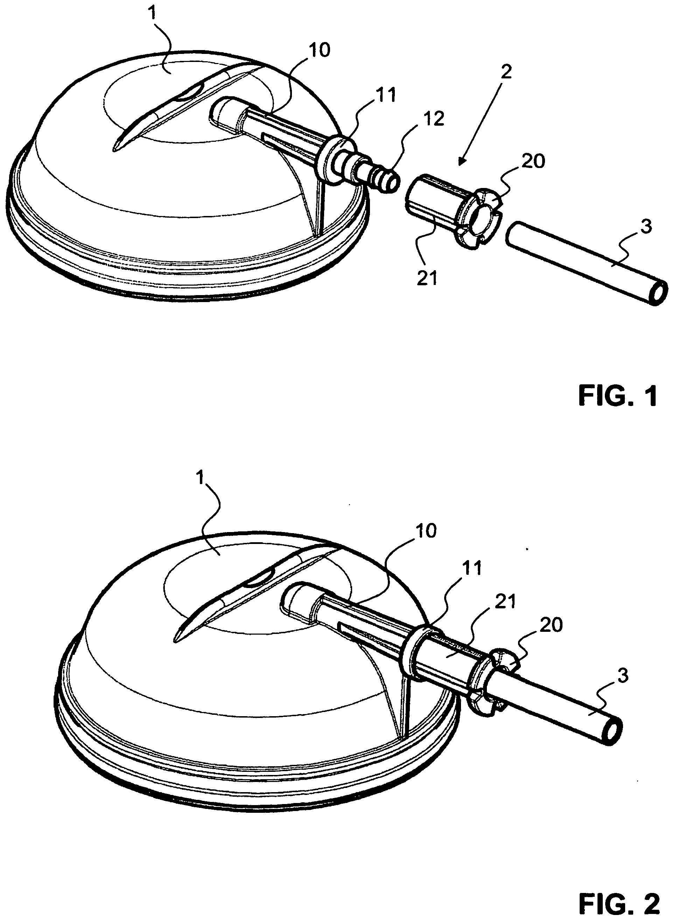

Die in den Figuren 1 bis 3 dargestellte Membrankappe 1 weist eine üblicherweise steife schüsselförmige Haube mit einer inneren Kavität 14 auf. Sie wird auf eine Pumpmembran einer Saugpumpe aufgesetzt und dicht mit ihr verbunden. Die Kavität 14 ist über eine in einem Anschlussstutzen 10 verlaufende Leitung 15 nach aussen verbunden. Der Anschlussstutzen 10 ist fest mit der Membrankappe 1 verbunden. Hier ist er einstückig mit ihr aus Kunststoff im Spritzgussverfahren hergestellt. Er ist vorzugsweise formsteif ausgebildet.

Das der Membrankappe 1 vorstehende freie Ende 12 des Anschlussstutzens 10 dient zur Aufnahme eines ersten Endes eines Saugschlauchs 3, welcher in den Figuren nur als kleiner Abschnitt dargestellt ist. Der Saugschlauch 3 ist über dieses freie Ende 12 gestülpt oder geschoben.

Über den Saugschlauch 3 und dem freien Ende 12 des Anschlussstutzens 10 ist eine Hülse 2 geschoben, welche mit ihrem schlauchfemen Ende an einem äusseren, nach aussen vorstehenden Flansch 11 des Anschlussstutzens 10 ansteht. Dies ist in Figur 2 erkennbar. Die Hülse 2 weist einen hohlzylinderförmigen Grundkörper 21 und einen daran am anschlussstutzenfernen Ende angeformten, radial nach aussen vorstehenden Schutzring oder Flansch 20 auf. Sie ist ebenfalls vorzugsweise aus einem Kunststoff im Spritzgussverfahren hergestellt.

Der Saugschlauch 3 ist an seinem zweiten Ende mit einer hier nicht dargestellten Brusthaube verbunden, welche zur Auflage auf eine menschliche Mutterbrust dient. Die Verbindung des Saugschlauchs mit der Brusthaube kann lösbar oder fest sein. Im letzteren Fall ist eine zerstörungsfreie Trennung nicht möglich.

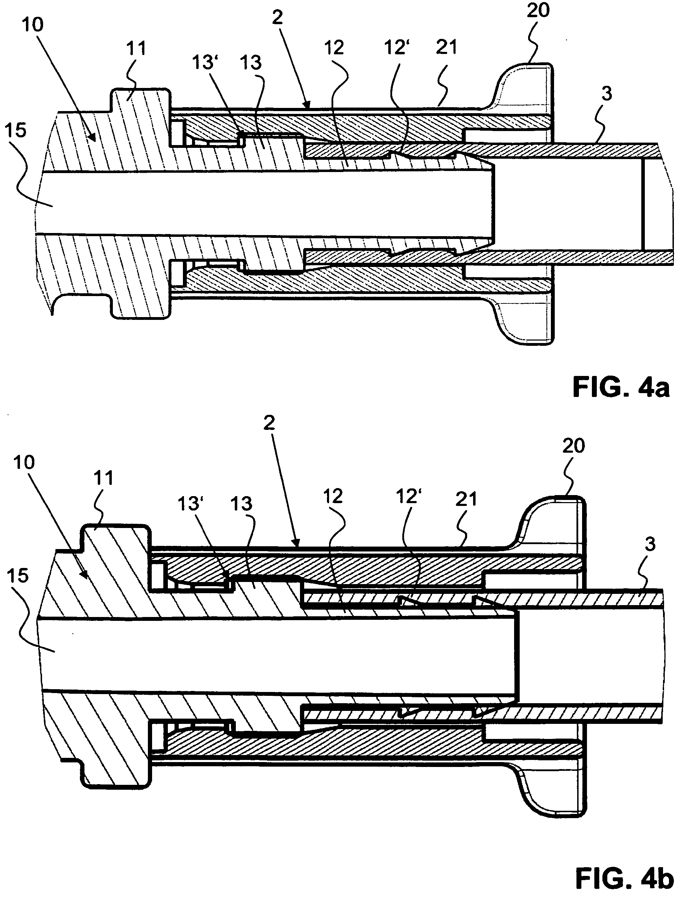

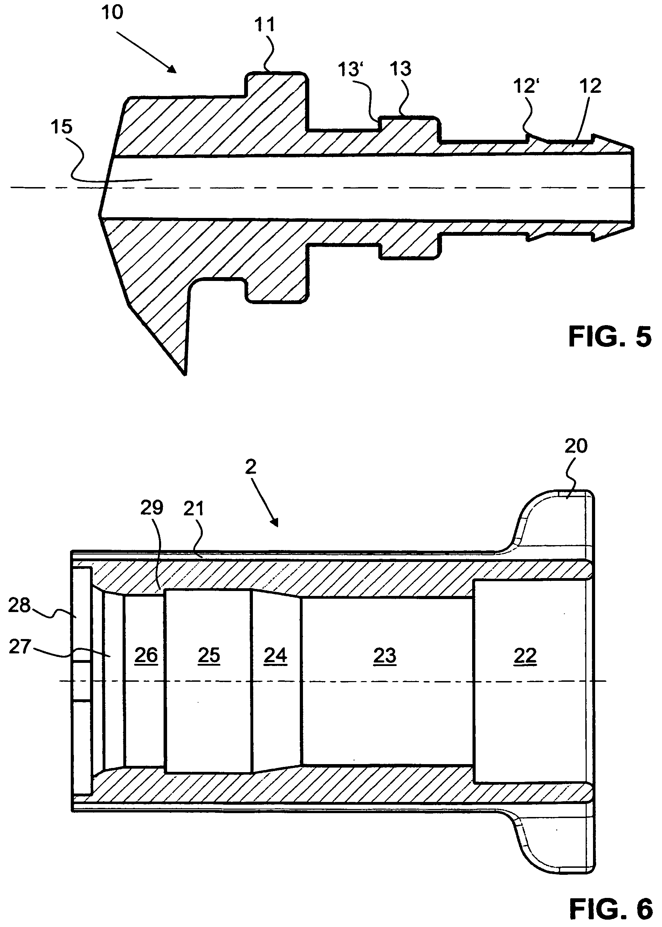

In den Figuren 4 bis 6 sind der Anschlussstutzen 10, die Hülse 2 und die Schnappverschluss-Verbindung dieser zwei Teile detailliert dargestellt. Der Anschlussstutzen 10 weist, wie in Figur 5 dargestellt ist, eine sich in Längsrichtung sprunghaft ändernde Aussenkontur auf. Entsprechend sprunghaft ändert sich eine Innenkontur der Hülse 2 in deren Längsrichtung, wie dies Figur 6 zeigt. Dadurch lässt sich eine nur durch Zerstörung lösbare Verbindung zwischen Anschlussstutzen 10 und Hülse 2 schaffen.

Der Anschlussstutzen gemäss Figur 5 ist an seinem freien schlauchseitigen Ende 12 mit Dichtrippen 12' versehen, welche vorzugsweise am schlauchfemen Ende scharfkantig bzw. schräg abfallend, insbesondere steil abfallend ausgebildet sind. Der Schlauch 3 lässt sich über das freie Ende 12 schieben und ist dank den Rippen 12' dichtend und fest gehalten, wie dies Figur 4 zeigt. Der Schlauch 3 lässt sich dabei maximal bis zu einem radial nach aussen vorstehenden inneren Flansch 13 des Anschlussstutzens 10 schieben. Dieser innere Flansch 13 weist einen kleineren Durchmesser auf als der äussere Flansch 11. Er bildet zumindest zum äusseren Flansch 11 hin, also an seiner schlauchfernen Seite, eine scharfe und/oder rechtwinklige Kante 13'.

Die Hülse gemäss Figur 6 weist eine innere Kontur mit mehreren, hier sieben Bereichen 22-28 auf, welche über Stufen ineinander übergehen und teilweise unterschiedliche innere Durchmesser aufweisen. Eine umlaufende Stufe oder Anschlagkante 29 zwischen zwei Bereichen, hier zwischen dem vierten und dem fünften Bereich, 25, 26, ist ebenfalls rechtwinklig und/oder scharfkantig ausgebildet. Dabei gehen die Bereiche 25, 26 in Richtung zum schlauchseitigen Ende hin von einem kleineren Innendurchmesser zu einem grösseren Innendurchmesser über.

Die Hülse 2 lässt sich nun zuerst über den Schlauch 3 und, nachdem der Schlauch 3 über den Anschlussstutzen 10 geschoben ist, ebenfalls über diesen pressen oder schieben. Dabei gleitet er mit seiner Stufe 29 über den inneren Flansch 13, bis er mit seiner schlauchfernen Stirnfläche am äusseren Flansch 11 ansteht. Ein Zurückziehen der Hülse 2 ist nun nicht mehr möglich, da ihre Kante 29 an der Kante 13' des Stutzens 10 ansteht. Die Verbindung lässt sich somit nur noch durch Zerstörung von Hülse 2 und/oder Anschlussstutzen 10 lösen.

Wie in Figur 4a erkennbar ist, dient die Hülse 2 bezüglich des Schlauchs 3 als Fixierung oder Dichtung. Sie liegt somit unter Druck auf dem Schlauch 3 auf.

Es ist aber auch möglich, wie in den nicht erfindungsgemäßen Beispiel von Figur 4b erkennbar ist, dass die Hülse 2 bezüglich des Schlauchs 3 nicht als Fixierung oder Dichtung dient. Sie liegt in diesem Fall beispielsweise entweder möglichst druckfrei auf dem Schlauch 3 auf oder sie lässt sogar, wie hier dargestellt, zum Schlauch hin über die gesamte gemeinsame Länge einen Zwischenraum frei.

Die Hülse 2 dient zudem als Schutz gegen aussen und, wenn sie wie hier dargestellt, mit ihrem schlauchnahen Ende den Anschlussstutzen 10 überragt, als Abknickschutz für den Schlauch 3. Sie erhöht somit die Steifigkeit der Schlauchverbindung.

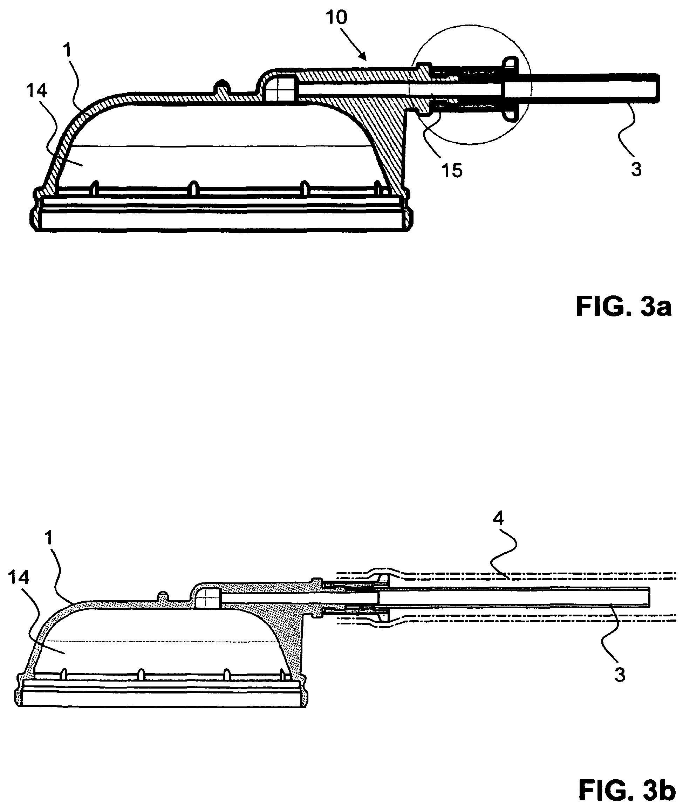

Erfindungsgemäss weist nun die Hülse 2 eine äussere Oberflächengestaltung auf, welche ein dichtendes Anliegen eines zweiten Saugschlauchs 4 auf der Hülse 2 verhindert, wie dies in Figur 3b erkennbar ist. Dies wird dadurch erreicht, dass die Aussenkontur der Hülse 2 mindestens über einen Teilbereich eine von einer Kreisform abweichende Form aufweist.

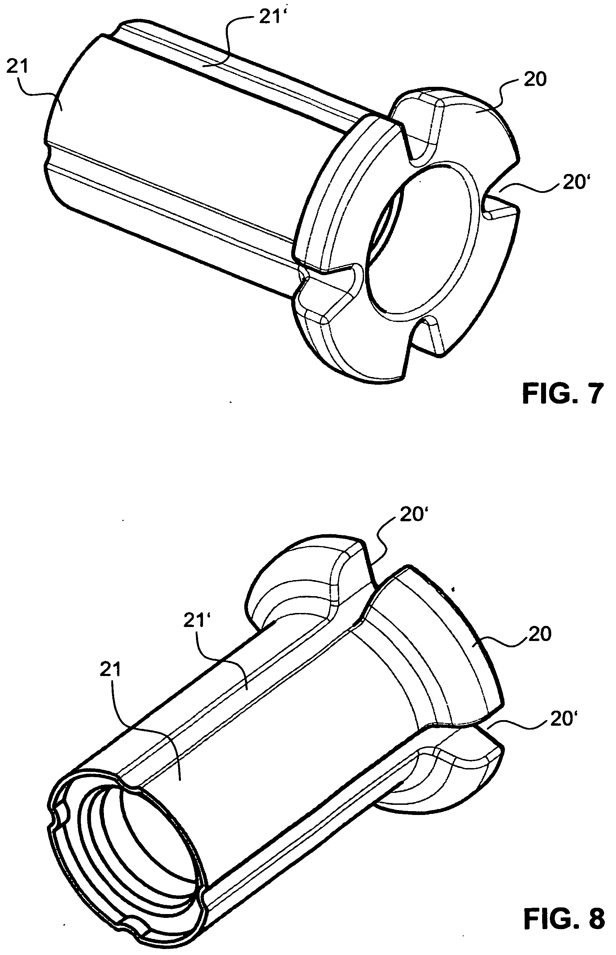

Erfindungsgemäß und wie in den Figuren 7 und 8 dargestellt weist die Hülse 2 hierfür den mindestens teilweise, hier ganz umlaufenden Flansch 20 auf, welcher rosettenartig ausgebildet ist und über seinen Umfang verteilt angeordnete Ausnehmungen oder Einbuchtungen 20' aufweist. Vorzugsweise ist seine schlauchferne Flanke gerundet bzw. langsam abfallend ausgebildet. Der Flansch 20 ist am anschlussstutzenfernen Ende der Hülse angeordnet.

Vorzugsweise sind im Grundkörper 21 der Hülse 2 zusätzlich Rippen und/oder Rillen 21' vorhanden. Vorzugsweise schliessen sie an die Vertiefungen oder Einbuchtungen 20' des Flansches 20 an und verlaufen parallel zur Längsachse des Grundkörpers 21. Anstelle oder zusätzlich zu den Rippen und Rillen 21' können auch Noppen oder Mulden oder andere Arten von Vertiefungen und/oder Erhebungen vorhanden sein. Im hier dargestellten Beispiel sind achsparallel verlaufende Rillen 21' vorhanden, welche an die Vertiefungen 20' anschliessen.

Die erfindungsgemässe Vorrichtung lässt sich nun vorzugsweise in einer Verbindungseinheit einer Brustpumpe zum Abpumpen von menschlicher Muttermilch einsetzen, wobei die Einheit diese Vorrichtung und den Saugschlauch umfasst, welcher mit einer Brusthaube verbindbar ist. Diese Verbindung mit der Brusthaube kann wiederum lösbar oder nur durch Zerstörung lösbar sein. Im zweiten Fall bildet die Brusthaube mit der genannten Verbindungseinheit eine Saugeinheit. Ferner kann auch eine an die Brusthaube angeschlossene Milchflasche oder ein anderer Milchauffangbehälter Bestandteil der Saugeinheit sein.

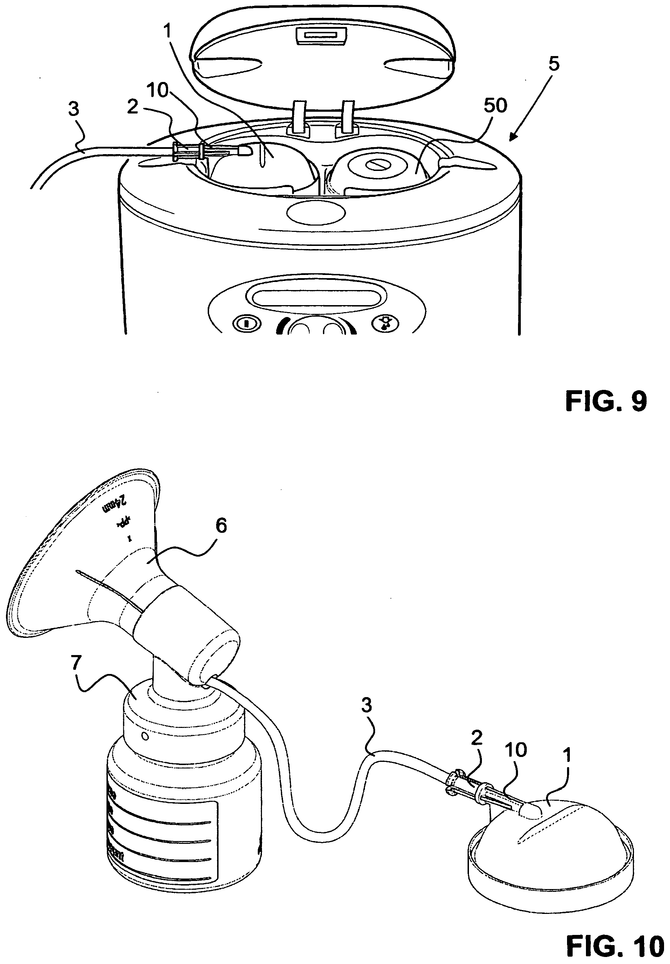

Alternativ dazu oder zusätzlich kann die Verbindungseinheit brustpumpenseitig mit einer Membrankappe verbindbar sein. Figur 9 zeigt einen oberen Teil einer Brustpumpe 5 mit zwei neben einander angeordneten Pumpenmembranen 50. Eine davon ist von einer Membrankappe 1 überdeckt, an welcher ein Saugschlauch 3 befestigt ist.

Ist, wie hier dargestellt, die Verbindung zwischen Membrankappe und Verbindungseinheit nur durch Zerstörung trennbar, so ist die Membrankappe wiederum Bestandteil der Verbindungseinheit.

Eine bevorzugte Kombination dieser Elemente führt zu einem Ersatzkit bzw. einer Saugeinheit für Brustpumpen, welche zumindest eine erfindungsgemässe Vorrichtung, eine mit dem Anschlussstutzen 10 einstückig oder nur durch Zerstörung lösbar verbundene Membrankappe 1, einen auf dem Anschlussstutzen 10 aufgeschobenen und mit der Hülse 2 überdeckten Saugschlauch 3 und eine am anderen Ende des Saugschlauchs 3 mit diesem lösbar oder nur über Zerstörung lösbare Brusthaube 6 umfasst. Die Brusthaube 6 kann zudem auf eine Milchflasche 7 oder auf einen Milchauffangbehälter aufgeschraubt sein. Ein entsprechendes Beispiel ist in Figur 10 dargestellt.

Somit lässt sich ein an diese erfindungsgemässe Vorrichtung dichtend angeschlossener Saugschlauch nicht ohne Zerstörung der Vorrichtung entfernen. Des Weiteren kann kein Schlauch mit einem grösseren Durchmesser dichtend auf die Hülse geschoben werden.

### Bezugszeichenliste

- 1: Membrankappe
- 10: Anschlussstutzen
- 11: äusserer Flansch
- 12: freies Ende
- 12': Dichtrippen
- 13: innerer Flansch
- 13': scharfe Kante
- 14: Kavität
- 15: Leitung

- 2: Hülse
- 20: Flansch
- 20': Einbuchtung
- 21: Grundkörper
- 21': Rille
- 22: erster Bereich
- 23: zweiter Bereich
- 24: dritter Bereich
- 25: vierter Bereich
- 26: fünfter Bereich
- 27: sechster Bereich
- 28: siebter Bereich
- 29: Anschlagkante

- 3: Saugschlauch
- 4: Ersatzschlauch

- 5: Brustpumpe
- 50: Pumpenmembran

- 6: Brusthaube

- 7: Milchflasche

## Patentansprüche

1. Vorrichtung zum Anschliessen eines Saugschlauchs (3), wobei die Vorrichtung einen Anschlussstutzen (10) aufweist, welcher derart ausgebildet ist, dass der Saugschlauch (3) dichtend über einen Bereich des Anschlussstutzens (10) aufschiebbar ist, wobei die Vorrichtung ferner eine Hülse (2) umfasst, welche über den genannten Bereich des Anschlussstutzens (10) aufschiebbar ist und diesen mindestens teilweise überdeckt, wobei zwischen Hülse (2) und Anschlussstutzen (10) Raum für den Saugschlauch (3) verbleibt, wobei die Hülse (2) einen Grundkörper (21) mit einem äusseren Durchmesser und einen am Grundkörper (21) angeformten äusseren Flansch (20) mit einem grösseren Durchmesser als der genannte Durchmesser aufweist, wobei der Flansch (20) am anschlussstutzenfernen Ende der Hülse (2) angeordnet ist, wobei die Hülse (2) auf ihrer Innenseite eine umlaufende Anschlagkante (29) aufweist und dass der Anschlussstutzen (10) einen radial nach aussen vorstehenden ersten Flansch (13) aufweist, **dadurch gekennzeichnet, dass** der erste Flansch (13) in Richtung entgegen einer Aufschieberichtung der Hülse (2) an dieser Anschlagkante (29) ansteht und dass die umlaufende Anschlagkante (29) und der radial nach aussen vorstehende erste Flansch (13) beide rechtwinklig und/oder scharfkantig ausgebildet sind, sodass die Verbindung zwischen Hülse (2) und Anschlussstutzen (10) nur durch Zerstörung mindestens eines dieser zwei Teile trennbar ist, und dass der Flansch (20) mindestens teilweise umlaufend ausgebildet ist und eine von einer Kreisform abweichende Aussenkontur aufweist, wobei der äussere Flansch (20) rosettenartig geformt ist, sodass der Flansch (20) ein dichtendes Anliegen eines Ersatzschlauchs auf der Hülse (2) verhindert.

2. Vorrichtung nach Anspruch 1, wobei die Verbindung zwischen Hülse (2) und Anschlussstutzen (10) nur durch Zerstörung beider Teile trennbar ist.

3. Vorrichtung nach Anspruch 1, wobei der Flansch (20) Vertiefungen (20') aufweist, an welche Vertiefungen (21') des Grundkörpers (21) anschliessen.

4. Vorrichtung nach einem der Ansprüche 1 bis 3, wobei die Anschlagkante (29) der Hülse (2) und der erste Flansch (13) des Anschlussstutzens (10) einen Schnappverschluss bilden.

5. Vorrichtung nach einem der Ansprüche 1 bis 4, wobei die Hülse (2) eine Durchgangsöffnung aufweist, welche über ihre Länge mehrfach ihren Innendurchmesser ändert.

6. Vorrichtung nach einem der Ansprüche 1 bis 5, wobei der Anschlussstutzen (10) ein freies Ende (12) mit Dichtrippen (12') zur dichtenden Aufnahme des Saugschlauchs (3) aufweist.

7. Vorrichtung nach einem der Ansprüche 1 bis 6, wobei die Hülse (2) im Wesentlichen hohlzylinderförmig ausgebildet ist und auf ihrer äusseren Oberfläche Vertiefungen (20') und/oder Erhöhungen aufweist, wobei diese Vertiefungen (20') und/oder Erhöhungen vorzugsweise parallel zur ihrer Längsachse angeordnet sind.

8. Vorrichtung nach einem der Ansprüche 1 bis 7, wobei die Hülse (2) und der Anschlussstutzen (10) aus Kunststoff gefertigt sind.

9. Vorrichtung nach einem der Ansprüche 1 bis 8, wobei der Anschlussstutzen (10) einen radial nach aussen vorstehenden zweiten Flansch (11) aufweist, welcher als Anschlag für die Hülse (2) dient.

10. Verbindungseinheit einer Brustpumpe zum Abpumpen von menschlicher Muttermilch, wobei die Verbindungseinheit eine Vorrichtung gemäss einem der Ansprüche 1 bis 9 und einen Saugschlauch (3) umfasst, welcher mit einem ersten Ende an dieser Vorrichtung angeschlossen ist.

11. Verbindungseinheit nach Anspruch 10, wobei die Vorrichtung brustpumpenseitig mit einer eine Pumpenmembran (50) schützenden und von der Pumpenmembran (50) entfernbaren Membrankappe (1) verbindbar ist, wobei der Anschlussstutzen (10) vorzugsweise so mit der Membrankappe (1) verbunden ist, dass er nur durch Zerstörung von dieser entfernbar ist.

12. Verbindungseinheit nach Anspruch 11, wobei der Anschlussstutzen (10) einstückig an der Membrankappe (1) angeformt ist.

13. Verbindungseinheit nach einem der Ansprüche 11 oder 12, wobei die Membrankappe (1) aus Kunststoff gefertigt ist.

14. Saugeinheit einer Brustpumpe zum Abpumpen von menschlicher Muttermilch, wobei die Saugeinheit eine Verbindungseinheit gemäss den Ansprüchen 12 bis 13 und eine Brusthaube (6) zur Anlage an eine Mutterbrust umfasst, wobei der Saugschlauch (3) an einem zweiten Ende mit der Brusthaube (6) verbunden ist.

## Claims

1. A device for connecting a suction tube (3), wherein the device has a connector piece (10) which is designed in such a way that the suction tube (3) can be pushed sealingly over an area of the connector piece (10), wherein the device further comprises a sleeve (2) which can be pushed over said area of the connector piece (10) and at least partially covers the latter, leaving room for the suction tube (3) between sleeve (2) and connector piece (10), wherein the sleeve (2) has a main body (21) with an external diameter and has an outer flange (20) which is formed integrally on the main body (21) and which has a diameter greater than said external diameter, wherein the flange (20) is arranged on that end of the sleeve (2) directed away from the connector piece, wherein the sleeve (2) has a circumferential abutment edge (29) on its inner face, and the connector piece (10) has a radially outwardly protruding first flange (13), **characterized in that** the first flange (13) bears against this edge (29) in the direction counter to a direction of pushing-on of the sleeve (2), and that the circumferential abutment edge (29) and the raidally oputwardly protruding first flange (13) both have a right-angled and/or sharp-edged design, so that the connection between sleeve (2) and connector piece (10) can be separated only by destroying at least one of these two parts, and that the flange (20) is at least partial circumferential and has an outer contour deviating from a circle shape, wherein the outer flange (20) has a rosette-like shape, so that the flange (20) prevents a replacement suction tube from bearing sealingly on the sleeve (2).

2. The device as claimed in claim 1, wherein the connection between sleeve (2) and connector piece (10) can be separated only by destroying both parts.

3. The device as claimed in claim 1, wherein the flange (20) has depressions (20'), which are adjoined by depressions (21') of the main body (21).

4. The device as claimed in one of claims 1 through 3, wherein the abutment edge (29) and the first flange (13) form a snap-fit closure.

5. The device as claimed in one of claims 1 through 4, wherein the sleeve (2) has a through-opening, which changes its internal diameter several times along its length.

6. The device as claimed in one of claims 1 through 5, wherein the connector piece (10) has a free end (12) with sealing ribs (12') for sealingly receiving the suction tube (3).

7. The device as claimed in one of claims 1 through 6, wherein the sleeve (2) has a substantially hollow cylindrical shape and has depressions (20') and/or elevations on its outer surface, wherein the depressions (20') and/or elevations are arranged preferably parallel to its longitudinal axis.

8. The device as claimed in one of claims 1 through 7, wherein the sleeve (2) and the connector piece (10) are made of plastic.

9. The device as claimed in one of claims 1 through 8, wherein the connector piece (10) has a radially outwardly protruding second flange (11) which serves as an abutment for the sleeve (2).

10. A connection unit of a breastpump for pumping off human breastmilk, wherein the connection unit comprises a device according to one of claims 1 through 9 and a suction tube (3) that is connected via a first end to this device.

11. The connection unit as claimed in claim 10, wherein the device can be connected at the breastpump end to a diaphragm cap (1) that protects a pump diaphragm (50) and that can be removed from the pump diaphragm (50), wherein the connector piece (10) is preferably connected to the diaphragm cap (1) such that it can be removed only by destroying the latter.

12. The connection unit as claimed in claim 11, wherein the connector piece (10) is formed integrally on the diaphragm cap (1).

13. The connection unit as claimed in one of claims 11 through 12, wherein the diaphragm cap (1) is made of plastic.

14. A suction unit of a breastpump for pumping off human breastmilk, wherein the suction unit comprises a connection unit according to claims 12 through 13 and a breastshield (6) for placing on a breast, wherein the suction tube (3) is connected at a second end to the breastshield (6).

## Revendications

1. Dispositif de raccordement d'un conduit souple de succion (3), le dispositif présentant une tubulure de raccordement (10) qui est réalisée de telle sorte que le conduit souple de succion (3) puisse être enfoncé de manière hermétique par-dessus une région de la tubulure de raccordement (10), le dispositif comprenant en outre une douille (2) qui peut être enfoncée sur ladite région de la tubulure de raccordement (10) et qui recouvre celle-ci au moins en partie, un espace subsistant entre la douille (2) et la tubulure de raccordement (10) pour le conduit souple de succion (3), la douille (2) présentant un corps de base (21) avec un diamètre extérieur et une bride extérieure (20) avec un plus grand diamètre que ledit diamètre, façonnée sur le corps de base (21), la bride (20) étant disposée à l'extrémité de la douille (2) éloignée de la tubulure de raccordement, la douille (2) présentant au niveau de son côté intérieur une arête de butée périphérique (29) et la tubulure de raccordement (10) présentant une première bride (13) saillant radialement vers l'extérieur, **caractérisé en ce que** la première bride (13) bute contre cette arête de butée (29) dans la direction opposée à une direction d'enfoncement de la douille (2), et **en ce que** l'arête de butée périphérique (29) et la première bride (13) saillant radialement vers l'extérieur sont toutes deux réalisées à angle droit et/ou avec des arêtes vives, de sorte que la connexion entre la douille (2) et la tubulure de raccordement (10) ne puisse être séparée que par destruction d'au moins l'une de ces deux parties et **en ce que** la bride (20) est réalisée sous forme au moins en partie périphérique et présente un contour extérieur s'écartant d'une forme circulaire, la bride extérieure (20) étant formée en forme de rosette, de telle sorte que la bride (20) empêche une application hermétique d'un conduit souple de remplacement sur la douille (2).

2. Dispositif selon la revendication 1, dans lequel la connexion entre la douille (2) et la tubulure de raccordement (10) ne peut être séparée que par destruction des deux parties.

3. Dispositif selon la revendication 1, dans lequel la bride (20) présente des renfoncements (20') auxquels se raccordent des renfoncements (21') du corps de base (21).

4. Dispositif selon l'une quelconque des revendications 1 à 3, dans lequel l'arête de butée (29) de la douille (2) et la première bride (13) de la tubulure de raccordement (10) forment une fermeture par encliquetage.

5. Dispositif selon l'une quelconque des revendications 1 à 4, dans lequel la douille (2) présente une ouverture de passage dont le diamètre intérieur varie plusieurs fois sur sa longueur.

6. Dispositif selon l'une quelconque des revendications 1 à 5, dans lequel la tubulure de raccordement (10) présente une extrémité libre (12) avec des nervures d'étanchéité (12') pour recevoir hermétiquement le conduit souple de succion (3).

7. Dispositif selon l'une quelconque des revendications 1 à 6, dans lequel la douille (2) est réalisée essentiellement sous forme cylindrique creuse et présente sur sa surface extérieure des renfoncements (20') et/ou des rehaussements, ces renfoncements (20') et/ou rehaussements étant disposés de préférence parallèlement à son axe longitudinal.

8. Dispositif selon l'une quelconque des revendications 1 à 7, dans lequel la douille (2) et la tubulure de raccordement (10) sont fabriquées en plastique.

9. Dispositif selon l'une quelconque des revendications 1 à 8, dans lequel la tubulure de raccordement (10) présente une deuxième bride (11) faisant saillie radialement vers l'extérieur, laquelle sert de butée pour la douille (2).

10. Unité de connexion d'un tire-lait pour pomper du lait maternel humain, l'unité de connexion comprenant un dispositif selon l'une quelconque des revendications 1 à 9, et un conduit souple de succion (3) qui est raccordé par une première extrémité à ce dispositif.

11. Unité de connexion selon la revendication 10, dans laquelle le dispositif peut être connecté du côté du tire-lait à un capuchon de membrane (1) protégeant la membrane de pompe (50) et pouvant être enlevé de la membrane de pompe (50), la tubulure de raccordement (10) étant de préférence connectée au capuchon de membrane (1) de telle sorte qu'elle ne puisse être enlevée de celui-ci que par destruction.

12. Unité de connexion selon la revendication 11, dans laquelle la tubulure de raccordement (10) est façonnée d'une seule pièce sur le capuchon de membrane (1).

13. Unité de connexion selon l'une quelconque des revendications 11 ou 12, dans laquelle le capuchon de membrane (1) est fabriqué en plastique.

14. Unité de succion d'un tire-lait pour pomper du lait maternel humain, l'unité de succion comprenant une unité de connexion selon les revendications 12 à 13, et une téterelle (6) pour l'application contre un sein maternel, le conduit souple de succion (3) étant connecté à la téterelle (6) au niveau d'une deuxième extrémité.
